**Europäisches Patentamt**

**European Patent Office**

**Office européen des brevets**

(11) Veröffentlichungsnummer: **0 428 514 B1**

# EUROPÄISCHE PATENTSCHRIFT

(45) Veröffentlichungstag der Patentschrift: **11.08.93**

(21) Anmeldenummer: **89904066.1**

(22) Anmeldetag: **08.04.89**

(86) Internationale Anmeldenummer:
**PCT/EP89/00381**

(87) Internationale Veröffentlichungsnummer:
**WO 90/01696 (22.02.90 90/05)**

(51) Int. Cl.⁵: **G01N 33/18**

Die Akte enthält technische Angaben, die nach
dem Eingang der Anmeldung eingereicht wurden
und die nicht in dieser Patentschrift enthalten sind.

(54) **VERFAHREN UND VORRICHTUNGEN ZUR BESTIMMUNG OXIDIERBARER WASSERINHALTSSTOFFE EINER WÄSSRIGEN PROBENFLÜSSIGKEIT.**

(30) Priorität: **13.08.88 DE 3827578**

(43) Veröffentlichungstag der Anmeldung:
**29.05.91 Patentblatt 91/22**

(45) Bekanntmachung des Hinweises auf die
Patenterteilung:
**11.08.93 Patentblatt 93/32**

(84) Benannte Vertragsstaaten:
**AT BE CH DE FR GB IT LI LU NL SE**

(56) Entgegenhaltungen:
**EP-A- 0 114 780**

**Patent Abstracts of Japan, Vol.8, Nr. 51
(P-259)(1488), 8 March 1984**

**Patent Abstracts of Japan, Vol. 8, Nr. 58
(P-261)(1495), 16 March 1984**

(73) Patentinhaber: **SIEPMANN, Friedrich Wilhelm
Oberdorf 34
W-6114 Gross-Umstadt/Raibach(DE)**

Patentinhaber: **TEUTSCHER, Michael
Neue Strasse 7
W-6107 Reinheim/Überau(DE)**

(72) Erfinder: **SIEPMANN, Friedrich Wilhelm
Oberdorf 34
W-6114 Gross-Umstadt/Raibach(DE)**
Erfinder: **TEUTSCHER, Michael
Neue Strasse 7
W-6107 Reinheim/Überau(DE)**

(74) Vertreter: **Katscher, Helmut, Dipl.-Ing.
Bismarckstrasse 29
W-6100 Darmstadt (DE)**

Patent Abstracts of Japan, Vol. 6, Nr. 129
(P-128)(1007), 15 July 1982

Patent Abstracts of Japan, Vol. 10, Nr. 9
(P-420)(2066) 14 January 1986

EP 0 428 514 B1

**Beschreibung**

Die Erfindung betrifft Verfahren zur Bestimmung oxidierbarer Wasserinhaltsstoffe, insbesondere des chemischen Sauerstoffbedarfs (CSB) oder des Gehalts an organischem Kohlenstoff (TOC - total organic carbon) einer wässrigen Probenflüssigkeit durch Oxidation von darin gelösten organischen Stoffen, wobei die Probenflüssigkeit dosiert mit Verdünnungswasser verdünnt wird.

Verfahren zur CSB-Bestimmung von wässrigen Probenflüssigkeiten, insbesondere Abwasser, sind bekannt. Vor allem bei der Überwachung von Abwässern sowohl bei der Einleitung in Gewässer wie auch am Zulauf oder Ablauf von Klärwerken hat die Bestimmung des CSB-Wertes eine zunehmende Bedeutung erlangt.

Bei den bekannten naßchemischen Verfahren erfolgt die Oxidation der in der Probenflüssigkeit gelösten organischen Stoffe durch chemische Reaktionen mit zugesetzten Oxidationmitteln. Diese Oxidationmittel bzw. die sich ergebenden Reaktionsprodukte sind jedoch überwiegend giftig oder mindestens umweltschädlich, so daß ihre Entsorgung Schwierigkeiten bereitet. Außerdem können diese Verfahren nicht kontinuierlich durchgeführt werden.

Bei einem bekannten Verfahren zum Bestimmen der Menge an organischem Kohlenstoff in einer Probenflüssigkeit (DE-A-24 58 143) ist zwar eine kontinuierliche Arbeitsweise möglich. Dieses Verfahren, bei dem eine Bestimmung der Menge der gasförmigen Oxidationprodukte durchgeführt wird, ist aber sowohl vom Verfahrensablauf als auch von der erforderlichen Vorrichtung her sehr aufwendig.

Bei einem anderen bekannten Verfahren (DE-A-23 62 773) zur Bestimmung von organisch gebundenem Kohlenstoff in Wasser durch Oxidation organischer Substanzen wird ebenfalls das bei der Oxidation gebildete Kohlendioxid bestimmt. Als Oxidationmittel wird dort Sauerstoff oder Luft verwendet. Unter ultravioletter Bestrahlung erfolgt eine fotochemische Oxidation der organischen Substanzen, wobei ein Platinkatalysator vorgesehen ist, um das bei der Bestrahlung des Sauerstoffs entstehende Ozon zu zerstören, bevor es zu der Oxidation beitragen kann. Dieses Verfahren arbeitet nicht kontinuierlich und ist daher für eine Prozeßsteuerung oder automatische laufende Überwachung nicht geeignet. Außerdem ist der Verfahrensablauf verhältnismäßig aufwendig.

Bei einem bekannten Verfahren der eingangs genannten Gattung erfolgt eine elektrochemische Oxidation der organischen Stoffe in einer potentiostatischen Meßanordnung. Bei der Oxidation der gelösten organischen Stoffe an einer Elektrode werden Elektronen abgegeben.

Zwischen dieser Elektrode und einer Gegenelektrode fließt dadurch ein Strom in einer Meßlösung, die aus der Probenflüssigkeit, Verdünnungswasser und einem Elektrolyten besteht. Dieser Stromfluß stellt bei konstantgehaltenem Elektrodenpotential ein Maß für die Menge der oxidierten organischen Stoffe dar. Die Funktionsfähigkeit der Elektroden wird beeinträchtigt, wenn sich durch in der Probenflüssigkeit enthaltene Chemikalien Beläge bilden. Deshalb ist es erforderlich, die Elektrode in gewissen Zeitabständen zu regenerieren. Hierzu muß der Meßablauf unterbrochen und ein Regenerationselektrolyt zugeführt werden. Eine ununterbrochene kontinuierliche Arbeitsweise ist deshalb nicht möglich. Um die Notwendigkeit oder die Wirksamkeit eines Regenerationsvorgangs festzustellen, ist es erforderlich, eine standardisierte Kalibrierflüssigkeit zuzuführen. Die Wirksamkeit und damit die Meßgenauigkeit des bekannten Verfahrens hängt außerdem davon ab, wie stark die Neigung der gelösten organischen Stoffe zu einer elektrochemischen Oxidation ist. Hierfür ist die Aufrechterhaltung eines hohen elektrischen Potentials zwischen den Elektroden erforderlich, wodurch wiederum die Belagbildung verstärkt wird.

Bei einem bekannten Verfahren (Patent Abstracts of Japan, Band 8, Nr. 51, P 259 1488, 1984) wird der wässrigen Probenflüssigkeit (z.B. Abwasser) zwar neben Sauerstoff auch Ozon zugeführt. Hierbei ist es jedoch nicht möglich, den Restozongehalt oder den Ozonverbrauch zu bestimmen. Die Probenflüssigkeit wird auch nicht dosiert mit Verdünnungswasser verdünnt. Das bekannte Verfahren ist nicht geeignet, bei sehr kurzen Meßzeiten aussagekräftige Meßergebnisse zu liefern.

Aufgabe der Erfindung ist es, Verfahren zur Bestimmung oxidierbarer Wasserinhaltsstoffe, insbesondere des chemischen Sauerstofbedarfs (CSB) oder des Gehalts an organischem Kohlenstoff (TOC - total organic carbon) zu schaffen, die ohne die Verwendung von gefährlichen chemischen Substanzen und ohne die bei der elektrochemischen Oxidation auftretenden Schwierigkeiten rasch und mit ausreichend hoher Genauigkeit bei geringem Aufwand an Meßgeräten durchgeführt werden können.

Bei einem kontinuierlichen Verfahren wird diese Aufgabe erfindungsgemäß dadurch gelöst, daß der Probenflüssigkeit zur Oxidation Ozon zugeführt wird, daß nach einer Durchmischung und Oxidation der Restozongehalt oder der Ozonverbrauch bestimmt wird und daß die Ozonzugabe und/oder das Mischungsverhältnis Probenflüssigkeit/Verdünnungswasser so geregelt wird bzw. werden, daß sich ein vorgegebener konstanter Restozongehalt bzw. Ozonverbrauch einstellt. Die Ozonzugabe kann in die verdünnte Probenflüssigkeit oder in das Verdünnungswasser erfolgen.

3

Die Verwendung von Ozon als Oxidationmittel hat den besonderen Vorteil, daß Ozon ein sehr stark reagierendes Oxidationmittel ist, so daß alle mit dem Ozon in Berührung kommenden organischen Stoffe sehr rasch oxidiert werden. Die hierfür erforderliche Zeit ist im wesentlichen nur dadurch bedingt, daß die Probenflüssigkeit ausreichend durchmischt werden muß, um alle darin enthaltenen organischen Stoffe mit dem zugeführten Ozon in Berührung zu bringen.

Bei dem erfindungsgemäßen Verfahren können das Mischungsverhältnis von Probenflüssigkeit und Verdünnungswasser und ggf. der Ozoneintrag so geregelt werden, daß eine hohe Genauigkeit des Verfahrens erreicht wird. Die Genauigkeit hängt von der Kontaktwahrscheinlichkeit zwischen Ozon und den zu oxidierenden Inhaltsstoffen ab und ist durch das Mischungsverhältnis bestimmt.

Das Meßverfahren ist dadurch besonders stabil, daß immer auf einen konstanten Restozongehalt bzw. Ozonverbrauch geregelt wird. Da eine hohe Konzentration an gelösten organischen Stoffen eine lange Reaktionszeit bis zum Erreichen eines aussagekräftigen Restozongehaltes führen würde, liegt ein besonderer Vorteil der erfindungsgemäßen Verfahren darin, daß durch die Verdünnung eine wesentlich schnellere Reaktion der organischen Stoffe mit dem Ozon und damit kürzere Meßzeiten erreicht werden.

Vorzugsweise erfolgt die Ozonanreicherung des Verdünnungswassers und die Oxidation der Wasserinhaltsstoffe mit dem ozonangereicherten Verdünnungswasser in zwei getrennten Reaktionräumen, und die Ozonmessung wird mit zwei Ozonmeßvorrichtungen, eine am Ablauf des Ozonanreicherungsgefäßes und eine zweite am Ablauf des Reaktionsgefäßes durchgeführt. Durch eine solche Meßanordnung läßt sich eine verminderte Ozonanreicherung durch Veränderung des Verdünnungswassers oder anderer Einflüsse erfassen und ausgleichen.

Bei verminderten Anforderungen an die Analysesicherheit und -genauigkeit kann auf eine solche Trennung des Anreicherungs- und Reaktionsgefäßes verzichtet werden.

Bei verminderten Anforderungen an die Analysensicherheit und -genauigkeit kann die Messung des Ozon vereinfachend durch Messung des Redoxpotentials durchgeführt werden.

Die Bestimmung des Redoxpotentials zur Ermittlung des Ozongehalts einer wässrigen Flüssigkeit ist bekannt und stellt in einem eingegrenzten Meßbereich, der etwa zwischen 400 und 800 mV liegt, ein mit geringem Aufwand und hoher Zuverlässigkeit durchzuführendes Meßverfahren dar.

Die genannten Redoxpotentiale entsprechen etwa 0,05 bis 0,30 mg Ozon pro Liter in Leitungswasser. Durch die hohe Verdünnung werden Meßwerte erreicht, die denen von Leitungswasser ähnlich sind. Bei der technischen Ausführung des Erfindungsgedankens sollte das eingetragene Ozon nach Oxidation der Inhaltsstoffe und der parallelen Ausgasung bis auf den genannten Restozongehalt vermindert sein. Es muß daher durch apparative Ausführung eine "Kurzschlußströmung" des Ozons von der Ozoneinleitung zum Meßpunkt sicher vermieden werden. Das hierbei entstehende "Grundrauschen" grenzt den Meßbereich der Redoxpotential-Messung nach unten bei ca. 400 mV ab. Der obere Meßbereich wird dadurch begrenzt, daß sich bei Leitungswasser ohne oxidierbare Inhaltsstoffe bei Begasung mit einem ozonhaltigen Gas ein Redoxpotential zwischen 800 und 1000 mV, im Mittel um 900 mV, einstellt. Die beiden Grenzen zeigen auch, wie die Empfindlichkeit der Geräte eingestellt werden kann. Je höher der einzustellende oder zu erreichende Restozon-Wert gewählt wird, desto ähnlicher ist die Auslaufkonzentration des Reaktionsbehälters der des Verdünnungswassers und umso empfindlicher reagiert das Verfahren.

Gemäß einer Ausführungsform der Erfindung ist vorgesehen, daß der Ozonverbrauch durch direkte Ozonmessungen vor der Ozonzugabe und nach der Oxidation und Differenzbildung bestimmt wird.

Gemäß einer Variante der erfindungsgemäßen Verfahren ist vorgesehen, daß bei konstantgehaltener Ozonzugabe das Mischungsverhältnis Probenflüssigkeit/Verdünnungswasser so geregelt wird, daß sich der konstante Restozongehalt einstellt und daß dieses Mischungsverhältnis zur Bestimmung oxidierbarer Wasserinhaltsstoffe verwendet wird. Dieses Mischungsverhältnis läßt sich leicht und mit hoher Genauigkeit feststellen; es unterliegt nur geringen Störungseinflüssen, da der Restozongehalt jeweils auf einen konstanten Wert und somit stabile und gut reproduzierbare Verhältnisse eingestellt wird.

Gemäß einer anderen Variante der erfindungsgemäßen Verfahren ist vorgesehen, daß bei konstantgehaltenem Mischungsverhältnis Probenflüssigkeit/Verdünnungswasser die Ozonzugabemenge so dosiert wird, daß sich ein konstanter Restozongehalt einstellt, und daß diese Ozonzugabemenge zur Bestimmung oxidierbarer Wasserinhaltsstoffe verwendet wird. Auch die Ozonzugabemenge läßt sich in gerätetechnisch einfacher Weise und mit ausreichender Genauigkeit regeln, beispielsweise durch Einstellung der Spannung, die am Ozonerzeuger angelegt wird.

Eine weitere Variante der Erfindung betrifft ein nicht kontinuierlich arbeitendes Verfahren zur Bestimmung oxidierbarer Wasserinhaltsstoffe einer wässrigen Probenflüssigkeit durch Oxidation von darin gelösten organischen Stoffen, wobei die Probenflüssigkeit vor der Oxidation dosiert mit Verdünnungswasser verdünnt wird und die Bestimmung oxidierbarer Wasserinhaltsstoffe in diskreten Einzelmessungen erfolgt. Dieses erfindungsgemäße Vefahren ist dadurch gekennzeichnet, daß die Ozonzugabe in einer dosierten Menge

4

erfolgt und daß die Zeitspanne bis zum Erreichen des vorgegebenen konstanten Restozongehalts bestimmt wird und ein Maß für die oxidierbaren Wasserinhaltsstoffe liefert.

Dieses Verfahren zeichnet sich durch einen besonders geringen apparativen Aufwand und eine einfache Meßdurchführung aus. Hierbei wird eine vorgegebene Menge von verdünnter Probenflüssigkeit mit einer ebenfalls vorgegebenen Menge von Ozon unter gleichbleibenden Mischungsbedingungen gemischt, wobei der Restozongehalt bzw. Ozonverbrauch ständig bestimmt wird. Die Zeit, die bis zum Erreichen eines vorgegebenen Restozongehalts benötigt wird, dient hierbei als Maß für die oxidierbarer Wasserinhaltsstoffe, insbesondere des chemischen Sauerstoffbedarfs (CBS) oder des Gehalts an organischem Kohlenstoff (TOC - total organic carbon).

Je höher die Konzentration an gelösten organischen Stoffen ist, desto länger ist die Reaktionszeit bis zum Erreichen eines vorgegebenen Restozongehalts. Dies ist bedingt durch den erforderlichen Eintrag des Ozons und die Notwendigkeit des Kontaktes mit den organischen Stoffen.

In jedem Fall wird für die Messung der oxidierbaren Inhaltsstoffe durch Zugabe von Ozon und Messung des Restozons ein weitgehender Abbau dieser Stoffe angestrebt, beispielsweise bis zu einer "technischen Abbaugrenze" von 10 % Restgehalt, da die Bedingungen dann stabiler sind.

Bei einer nicht kontinuierlich arbeitenden Variante des Erfindungsgedankens wird die Probenflüssigkeit vor der Oxidation dosiert, mit Verdünnungswasser verdünnt und kontinuierlich mit einer dosierten Ozonmenge pro Zeiteinheit versetzt und die Zeitspanne bis zum Erreichen eines vorgegebenen Restozon-Wertes gemessen.

Erfindungsgemäß sind folgende Verfahrensvarianten möglich:

a) Regelung der Mischpumpen nach einem konstanten Restozongehalt;

b) Regelung des Ozoneintrags bei konstanter Verdünnung nach einem konstanten Restozongehalt;

c) Messung der Zeitspanne bis zur Oxidation der Inhaltsstoffe und Nachweis von Restozon.

Jede der Varianten hat ihren wirtschaftlich interessanten Einsatzbereich. So ist mit Variante a) in einem großen Meßbereich, der nur durch das Verhältnis der Mischpumpen begrenzt ist, ein Meßwert mit hoher Genauigkeit zu erzeugen. Durch die Regelung der Pumpen wird immer eine geringe, konstante Konzentration oxidierbarer Inhaltsstoffe in den Reaktionsbehälter eingetragen. Bei guter Vermischung und ausreichender Aufenthaltszeit im Reaktionsgefäß von beispielsweise 5 Minuten bei einem Volumenstrom von 500 ml/Min und einem Restozon von 80% der eingetragenen Ozonmenge wird (gemäß Empfindlichkeitseinstellung) die gewünschte geringe Konzentration oxidierbarer Stoffe im Reaktionsbehälter durch eine Ozonbeladung von vorzugsweise weniger als 0,5 gewährleistet. Das garantiert eine konstante Aufenthaltszeit der Reaktanten im Reaktionsbehälter unabhängig von der Ausgangskonzentration der zu untersuchenden Flüssigkeit. Die hohe Verdünnung verhindert eine weitgehende Schaumbildung und garantiert so beim Eintrag des Ozons in einem Gasgemisch einen störungsfreien Betrieb.

Die Variante b) ist technisch einfacher zu realisieren als die Variante a). Der Meßbereich sollte hier in engeren Grenzen schwanken, wie etwa bei einer CSB-Messung am Auslauf einer Kläranlage, da bei konstanter Aufenthaltszeit im Reaktionsbehälter unterschiedliche CSB-Konzentrationen auf einen gleichen Endabbaugrad oxidiert werden sollten. Dieser Nachteil läßt sich teilweise mathematisch kompensieren und/oder durch eine größere Sicherheit in der Aufenthaltszeit im Reaktionsbehälter ausgleichen.

Die Variante c) ist mit einer technisch wesentlich einfacheren Geräteausführung für den nicht kontinuierlichen Betrieb durchführbar.

Vorteilhafte Ausgestaltungen der erfindungsgemäßen Verfahren sowie Vorrichtungen zur Durchführung dieser Verfahren sind Gegenstand von Unteransprüchen.

Nachfolgend werden erfindungsgemäße Verfahren und Vorrichtungen zur Durchführung dieser Verfahren näher erläutert, die in der Zeichnung dargestellt sind. Es zeigt:

Fig. 1 in schematischer Darstellungsweise eine Vorrichtung zur Durchführung der erfindungsgemäßen kontinuierlichen Verfahren,

Fig. 2 ebenfalls in schematischer Darstellungsweise eine Vorrichtung zur Durchführung eines nichtkontinuierlich arbeitenden erfindungsgemäßen Verfahrens,

Fig. 3 ein Diagramm, in dem das Redoxpotential über den Ozongehalt aufgetragen ist,

Fig. 4 ebenfalls in schematischer Darstellungsweise eine weitere Vorrichtung zur Durchführung des erfindungsgemäßen Verfahrens und

Fig. 5 ein Diagramm, in dem die Ozonbeladung über dem Ozonverbrauch aufgetragen ist.

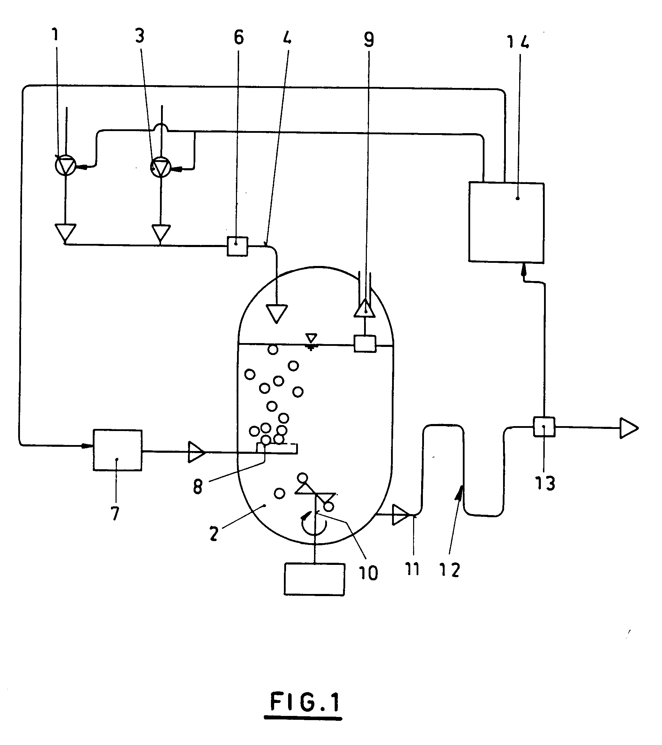

Bei der in Fig. 1 gezeigten Vorrichtung zur CSB-Bestimmung wird die Probenflüssigkeit, beispielsweise Abwasser, durch eine Dosierpumpe 1 einem geschlossenen Reaktionsbehälter 2 zugeführt. Zugleich wird durch eine Dosierpumpe 3 Verdünnungswasser zugeführt. In der Zulaufleitung 4 des Reaktionsbehälters 2 ist als Ozonmeßeinrichtung eine das Redoxpotential des zugeführten Gemischs bestimmende Redoxpotential-Meßzelle 6 angeordnet.

Ein Ozonerzeuger 7, der über eine Einperleinrichtung 8 einen dosierbaren Mengenstrom von Ozon an den Reaktionsbehälter 2 liefert, bildet eine Oxidationeinrichtung.

Ein Entgasungsventil 9 ermöglicht den Austritt der Gasmenge, die in der Probenflüssigkeit nicht reagiert hat und nicht gelöst wurde.

Eine in Fig. 1 nur schematisch als Rührwerk dargestellte Durchmischungseinrichtung 10 dient dazu, den Inhalt des Reaktionsbehälters 2 zu durchmischen und etwaige Feststoffteilchen zu zerkleinern, die mit dem Abwasser eingetragen werden.

Die Probenflüssigkeit gelangt aus dem Reaktionsbehälter 2 in eine Behälterauslaßleitung 11, in der ein Rohrreaktor 12, beispielsweise in Form einer Rohrschlange, angeordnet ist. Am Ende des Rohrreaktors 12 befindet sich als Ozonmeßeinrichtung eine Redoxpotential-Meßzelle 13. Diese Redoxpotential-Meßzelle 13 mißt den Restozongehalt, der sich nach der Reaktionszeit einstellt, die durch den Mischvorgang im Reaktionsbehälter 2 und durch die Pfropfenströmung durch den Rohrreaktor 12 bestimmt ist. Der Rohrreaktor 12 dient dazu, eine Kurzschlußströmung von Ozon von der Einperleinrichtung 8 direkt zur Redoxpotential-Meßzelle 13 zu verhindern, weil dadurch das Meßergebnis verfälscht würde. Er stellt eine Mindestreaktionszeit sicher.

Das Meßergebnis der Redoxpotential-Meßzelle 13 wird einer Regeleinrichtung 14 zugeführt, die die Dosierpumpen 1, 3 und/oder den Ozonerzeuger 7 steuert.

Der Regelvorgang erfolgt beispielsweise in der Weise, daß der Ozoneintrag durch den Ozonerzeuger 7 konstantgehalten wird und daß das Mischungsverhältnis Probenflüssigkeit/Verdünnungswasser durch Steuerung der Dosierpumpen 1 und 3 so geregelt wird, daß sich an der Redoxpotential-Meßzelle 13 von Regelschwankungen abgesehen ein vorgegebenes konstantes Redoxpotential einstellt. Dieses vorgegebene konstante Redoxpotential wird im Bereich von 300 bis 800 mV, vorzugsweise bei ungefähr 700 mV gewählt.

Stattdessen können die Dosierpumpen 1 und 3 auch auf ein konstantes Verdünnungsverhältnis eingestellt werden. Die Regeleinrichtung 14 regelt dann den Ozoneintrag durch den Ozonerzeuger 7 so, daß sich an der Redoxpotential-Meßzelle 13 ebenfalls wieder das konstante Redoxpotential einstellt.

Da auch die Temperatur die Reaktion beeinflußt, wird zweckmäßigerweise die Temperatur der verdünnten Probenflüssigkeit bestimmt und beispielsweise bei 25°C konstantgehalten.

Die Carbonathärte des zugegebenen Verdünnungswassers bzw. der verdünnten Probenflüssigkeit ist von Einfluß auf die Reaktion und sollte deshalb bestimmt und konstant gehalten werden. Besonders zweckmäßig ist es, durch Enthärtung des Verdünnungswassers eine Carbonathärte von angenähert 0 einzustellen.

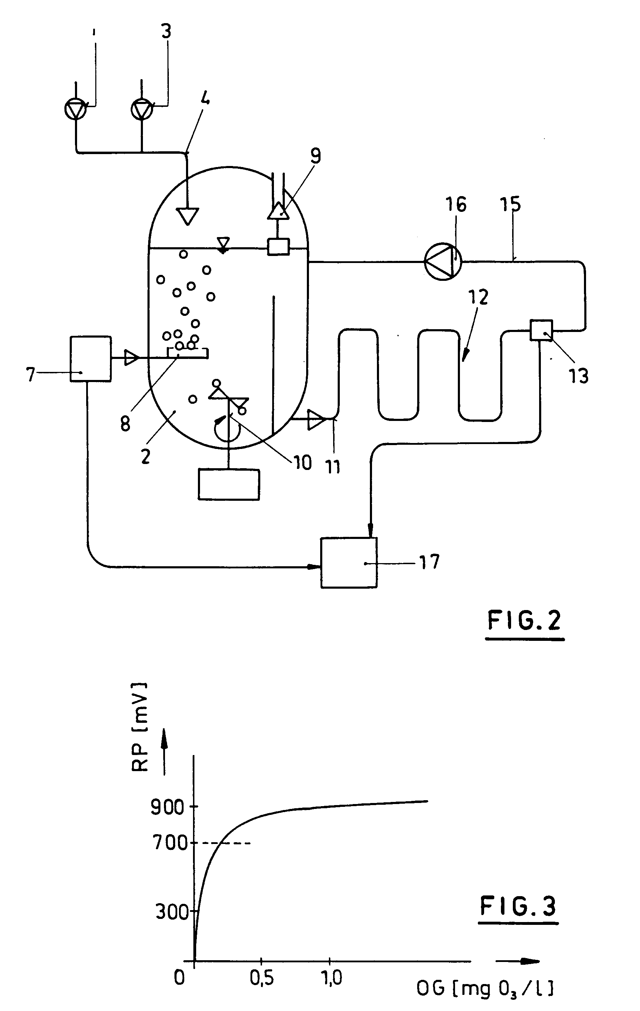

Eine andere Vorrichtung zur Durchführung eines abgewandelten Verfahrens ist in Fig. 2 gezeigt. Der Reaktionsbehälter 2 und weitere Teile sind hierbei ähnlich aufgebaut wie bei der Vorrichtung nach Fig. 1 und sind deshalb mit gleichen Bezugszeichen bezeichnet. Die in Fig. 2 gezeigte Vorrichtung unterscheidet sich von der Vorrichtung nach Fig. 1 im wesentlichen dadurch, daß die Befüllung des Reaktionsbehälters 2 nicht kontinuierlich, sondern chargenweise erfolgt. Die Probenflüssigkeit wird durch den Rohrreaktor 12 und die Redoxpotential-Meßzelle 13 durch eine Kreislaufleitung 15 mittels einer Umwälzpumpe 16 im Kreislauf wieder in den Reaktionsbehälter 2 zurückgeführt. Nachdem durch die Dosierpumpen 1 und 3 eine vorgegebene Mischung von Probenflüssigkeit und Verdünnungswasser in dosierter Menge in den Reaktionsbehälter 2 gegeben und durch den Ozonerzeuger 7 eine vorgegebene Ozonmenge eingespeist wurde, wird die Mischung durch die Umwälzpumpe 16 solange im Kreislauf geführt, bis sich an der Redoxpotential-Meßzelle 13 das vorgegebene Redoxpotential einstellt, das ebenfalls beispielsweise bei etwa 700 mV gewählt wurde. In einer Auswerteschaltung 17, die mit dem Ozonerzeuger und der Redoxpotential-Meßzelle 13 verbunden ist, wird die Zeitspanne zwischen der Ozoneinleitung und dem Erreichen des vorgegebenen Redoxpotential-Werts bestimmt und dient als Maß für den CSB.

Fig. 3 zeigt den Verlauf des Redoxpotentials RP über dem Ozongehalt OG.

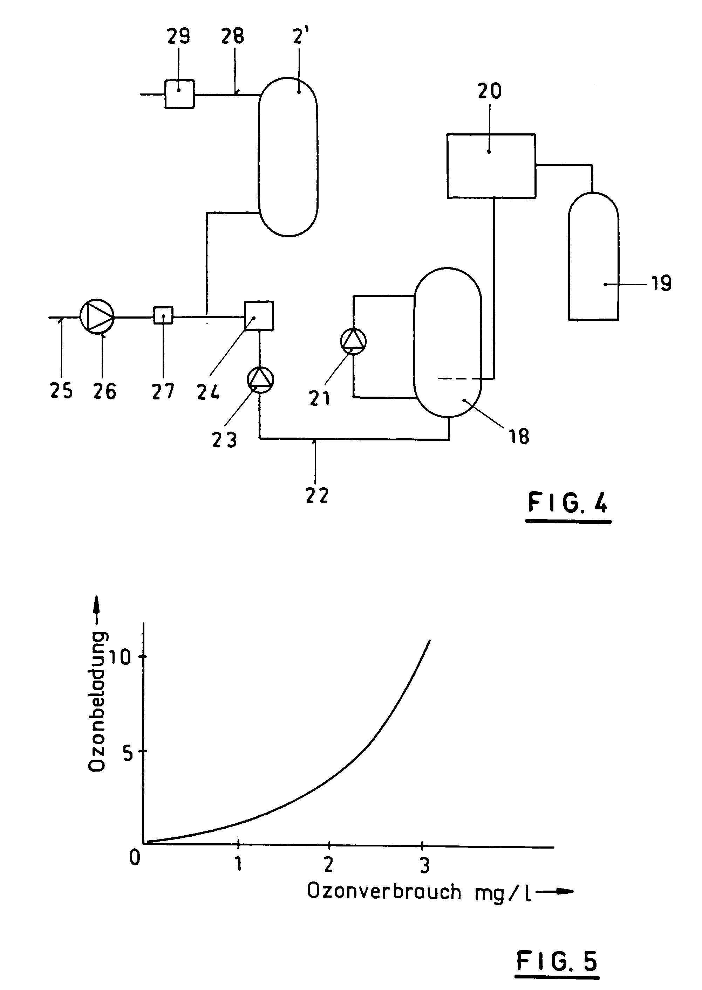

Bei der in Fig. 4 vereinfacht dargestellten Vorrichtung zur Durchführung des Verfahrens wird das Verdünnungswasser in einem Anreicherungs-Reaktionsbehälter 18 bereits vor der Durchmischung mit dem Abwasser mit Ozon angereichert. Hierzu ist ein mit einer Sauerstoffquelle 19 verbundener Ozonerzeuger 20 an den Reaktionsbehälter 18 angeschlossen.

Eine über eine Zuleitung und einer Rückleitung an den Reaktionsbehälter 18 angeschlossene Umwälzpumpe 21 sorgt für eine intensive Vermischung des Verdünnungswassers mit dem zugeführten Ozon. Eine Leitung 22 führt von dem Reaktionsbehälter 18 über eine Verdünnungswasserpumpe 23 und eine als Ozonmeßsonde ausgeführte Ozonmeßeinrichtung 24 in einen Reaktionsbehälter 2'.

In diesen Reaktionsbehälter 2' führt auch eine Leitung 25, durch die Abwasser als Probenflüssigkeit über eine Abwasserpumpe 26 und ein Magnetventil 27 zugeführt wird. Dabei erfolgt eine Vermischung des dosiert zugeführten Abwassers mit dem ebenfalls dosiert zugeführten, mit Ozon angereicherten Verdünnungswasser.

Im Reaktionsbehälter 2' erfolgt die Oxidation der Wasserinhaltstoffe des Abwassers durch das Ozon. In der Ausgangsleitung 28 des Reaktionsbehälters 2' ist als zweite Ozonmeßeinrichtung 29 eine Ozonmeßsonde angeordnet.

Durch Steuerung der Pumpen 23 und 26 und/oder des Ozongenerators 20 werden das Mischungsverhältnis Abwasser/Verdünnungswasser und/oder die Ozonzugabe so geregelt, daß sich an der Ozonmeßeinrichtung 29 ein vorgegebener konstanter Restozongehalt bzw. - durch die Differenzmessung an den Ozonmeßeinrichtungen 24 und 29 - ein vorgegebener konstanter Ozonverbrauch einstellt. Das dabei jeweils durch die Pumpen 23 und 26 eingestellte Mischungsverhältnis oder die am Ozonerzeuger 20 eingestellte Ozonzugabe stellen ein Maß für die oxidierbaren Wasserinhaltsstoffe des durch die Leitung 25 zugeführten Abwassers oder einer sonstigen Probenflüssigkeit dar.

Bei der Beschreibung der Ausführungsbeispiele wurde vereinfachend nur von einer CSB-Bestimmung gesprochen. Die Beispiele gelten aber allgemein für die Bestimmung oxidierbarer Wasserinhaltsstoffe, beispielsweise auch des TOC.

Bei dem geschilderten Verfahren kommt der Regelung des Mischungsverhältnisses von Probenflüssigkeit und Verdünnungswasser, ggf. in Verbindung mit einer Regelung des Ozoneintrag eine besondere Bedeutung zu. So hängt die Genauigkeit des Verfahrens ganz wesentlich von der Kontaktzeit und der Kontaktwahrscheinlichkeit zwischen Ozon und den zu oxidierenden Inhaltstoffen ab. Die Kontaktzeit wird durch die Dimensionierung des Reaktionsbehälters festgelegt. Die Kontaktwahrscheinlichkeit wird durch das Verhältnis von im Verdünnungswasser gelösten Ozon und Abwasserinhaltstoffen festgelegt. Dieses Verhältnis wird hier als Ozonbeladung wie folgt definiert:

$$\text{Ozonbeladung} = \frac{\text{CSB-Fracht (mg/Min)}}{\text{Ozon-Fracht (mg/Min)}}.$$

Durch Fig. 5 wird deutlich, daß mit Festlegung eines Restozongehaltes bzw. Ozonverbrauchs die Reaktionsbedingungen bei konstantem Reaktionsbehälter festgelegt sind und damit die Empfindlichkeit und der Meßbereich des Meßverfahrens.

Zum Erreichen einer hohen Genauigkeit ist die Festlegung einer "Ozonbeladung" auf einem Arbeitspunkt in einem Bereich kleiner 0,5 zu empfehlen.

Die Genauigkeit des Verfahrens ist damit nur durch den Regelbereich der Pumpen begrenzt. Durch die Erhöhung des Ozoneintrags kann der Meßbereich nach oben erweitert werden. Oberhalb dieses Bereiches ist die Messung bei verminderter Genauigkeit ebenfalls möglich.

**Patentansprüche**

1. Kontinuierliches Verfahren zur Bestimmung oxidierbarer Wasserinhaltsstoffe, insbesondere des chemischen Sauerstoffbedarfs (CSB) oder des Gehalts an organischem Kohlenstoff (TOC - total organic carbon) einer kontinuierlich zugeführten wässrigen Probenflüssigkeit durch Oxidation von darin gelösten organischen Stoffen, wobei die Probenflüssigkeit dosiert mit Verdünnungswasser verdünnt wird, dadurch gekennzeichnet, daß der Probenflüssigkeit zur Oxidation Ozon zugeführt wird, daß nach einer Durchmischung und Oxidation der Restozongehalt oder der Ozonverbrauch bestimmt wird, und daß die Ozonzugabe und/oder das Mischungsverhältnis Probenflüssigkeit/Verdünnungswasser so geregelt wird bzw. werden, daß sich ein vorgegebener konstanter Restozongehalt bzw. Ozonverbrauch einstellt.

2. Verfahren nach Anspruch 1, dadurch gekennzeichnet, daß die Probenflüssigkeit vor der Ozonzugabe mit dem Verdünnungswasser verdünnt wird.

3. Verfahren nach Anspruch 1, dadurch gekennzeichnet, daß das Verdünnungswasser mit Ozon angereichert und anschließend mit der Probenflüssigkeit vermischt wird.

4. Verfahren nach Anspruch 1, dadurch gekennzeichnet, daß der Restozongehalt durch Messung des Redoxpotentials bestimmt wird.

**5.** Verfahren nach Anspruch 1, dadurch gekennzeichnet, daß der Ozonverbrauch durch direkte Ozonmessungen vor der Ozonzugabe und nach der Oxidation und Differenzbildung bestimmt wird.

**6.** Verfahren nach Anspruch 1, dadurch gekennzeichnet, daß ein Verhältnis der oxidierbaren Wasserinhaltsstoffe, insbesondere des chemischen Sauerstoffbedarfs (CSB), zur Menge des zugegebenen Ozons von höchstens 0,5 eingehalten wird.

**7.** Verfahren nach Anspruch 3, dadurch gekennzeichnet, daß die Ozonanreicherung des Verdünnungswassers und die Oxidation in zwei getrennten Reaktionsräumen erfolgt und daß am Ablauf beider Reaktionsräume jeweils eine Ozonmessung erfolgt.

**8.** Verfahren nach Anspruch 1, dadurch gekennzeichnet, daß die Bestimmung des Restozongehalts in einem Bereich von 0,05 - 0,3 mg Ozon pro Liter erfolgt.

**9.** Verfahren nach Anspruch 1, dadurch gekennzeichnet, daß bei konstantgehaltener Ozonzugabe das Mischungsverhältnis Probenflüssigkeit/Verdünnungswasser so geregelt wird, daß sich ein konstanter Restozongehalt bzw. Ozonverbrauch einstellt und daß dieses Mischungsverhältnis zur Bestimmung oxidierbarer Wasserinhaltsstoffe verwendet wird.

**10.** Verfahren nach Anspruch 1, dadurch gekennzeichnet, daß bei konstantgehaltenem Mischungsverhältnis Probenflüssigkeit/Verdünnungswasser die Ozonzugabemenge so dosiert wird, daß sich ein konstanter Restozongehalt bzw. Ozonverbrauch einstellt, und daß diese Ozonzugabemenge zur Bestimmung oxidierbarer Wasserinhaltsstoffe verwendet wird.

**11.** Diskontinuierliches Verfahren zur Bestimmung oxidierbarer Wasserinhaltsstoffe einer wässrigen Probenflüssigkeit durch Oxidation von darin gelösten organischen Stoffen, wobei die Probenflüssigkeit dosiert mit Verdünnungswasser verdünnt wird und die Bestimmung oxidierbarer Wasserinhaltsstoffe in diskreten Einzelmessungen erfolgt, dadurch gekennzeichnet, daß der Probenflüssigkeit zur Oxidation Ozon zugeführt wird, daß nach einer Durchmischung und Oxidation der Restozongehalt bestimmt wird, daß die Ozonzugabe in einer dosierten Menge erfolgt und daß die Zeitspanne bis zum Erreichen des vorgegebenen konstanten Restozongehalts bestimmt wird und ein Maß für die oxidierbaren Wasserinhaltsstoffe liefert.

**12.** Verfahren nach Anspruch 4, dadurch gekennzeichnet, daß das Redoxpotential im Bereich von 300 bis 800 mV vorgegeben wird.

**13.** Vorrichtung zur Durchführung des Verfahrens nach einem der Ansprüche 1 bis 12 mit Dosierpumpen für die Probenflüssigkeit und das Verdünnungswasser, mindestens einem Reaktionsbehälter, einer Durchmischungseinrichtung, einer Oxidationseinrichtung und einer Behälterauslaßleitung, dadurch gekennzeichnet, daß die Oxidationseinrichtung ein Ozonerzeuger (7, 20) ist und daß in der Behälterauslaßleitung (11, 28) eine Ozonmeßeinrichtung (29) bzw. eine Redoxpotential-Meßzelle (13) angeordnet ist.

**14.** Vorrichtung nach Anspruch 13, dadurch gekennzeichnet, daß der Ozonerzeuger (20) mit einem Reaktionsbehälter (18) verbunden ist, in dem das Verdünnungswasser mit Ozon angereichert wird.

**15.** Vorrichtung nach Anspruch 13, dadurch gekennzeichnet, daß die Ozonmeßeinrichtung (13) mit einer Regeleinrichtung (14) für die Dosierpumpen (1, 3) der Probenflüssigkeit und/oder des Verdünnungswassers verbunden ist.

**16.** Vorrichtung nach Anspruch 15, dadurch gekennzeichnet, daß die Ozonmeßeinrichtung (13) mit einer Regeleinrichtung (14) für den Ozonerzeuger (7) verbunden ist.

**17.** Vorrichtung zur Durchführung des Verfahrens nach Anspruch 12 mit einem Reaktionsbehälter, einer Durchmischungseinrichtung und einer Oxidationseinrichtung, dadurch gekennzeichnet, daß die Oxidationseinrichtung ein Ozonerzeuger (7) mit einer Dosiereinrichtung ist und daß der Reaktionsbehälter (2) mit einer Ozonmeßeinrichtung (13) verbunden ist.

**18.** Vorrichtung nach Anspruch 17, dadurch gekennzeichnet, daß an den Reaktionsbehälter (2) eine Kreislaufleitung (15) angeschlossen ist, in der eine Umwälzpumpe (16) und die Ozonmeßeinrichtung (13) angeordnet sind.

**19.** Vorrichtung nach Anspruch 17 oder 18, dadurch gekennzeichnet, daß die Dosiereinrichtung der Oxidationseinrichtung (7) und die Ozonmeßeinrichtung (13) mit einer Auswerteschaltung (17) verbunden sind, in der die Zeitspanne zwischen der Ozoneinleitung und dem Erreichen eines vorgegebenen Wertes des Ozongehalts, des Ozonverbrauchs oder Redoxpotentials bestimmt wird.

## Claims

**1.** Continuous method for determining oxidisable substances contained in water, in particular the chemical oxygen demand (COD) or the total organic carbon (TOC) of a continuously supplied aqueous sample liquid by oxidation of organic substances dissolved therein, the sample liquid being diluted in a dosed manner with diluting water, characterised in that ozone is supplied to the sample liquid for oxidation, that after thorough mixing and oxidation, the residual ozone content or the ozone consumption is determined and that the addition of ozone and/or the mixing ratio of sample liquid/diluting water is/are controlled so that a predetermined, constant residual ozone content or ozone consumption is regulated.

**2.** Method according to Claim 1, characterised in that before the addition of ozone, the sample liquid is diluted with the diluting water.

**3.** Method according to Claim 1, characterised in that the diluting water is enriched with ozone and then mixed with the sample liquid.

**4.** Method according to Claim 1, characterised in that the residual ozone content is determined by measuring the redox potential.

**5.** Method according to Claim 1, characterised in that the ozone consumption is determined by direct ozone measurements before the addition of ozone and after the oxidation and formation of the difference.

**6.** Method according to Claim 1, characterised in that a ratio of the oxidisable substances contained in the water, in particular of the chemical oxygen demand (COD) with respect to the quantity of ozone added of at the most 0.5 is maintained.

**7.** Method according to Claim 3, characterised in that the ozone enrichment of the diluting water and the oxidation takes place in two separate reaction chambers and that respectively one ozone measurement takes place at the outlet of both reaction chambers.

**8.** Method according to Claim 1, characterised in that the determination of the residual ozone content takes place in a range of 0.05 - 0.3 mg ozone per litre.

**9.** Method according to Claim 1, characterised in that when the addition of ozone is kept constant, the mixing ratio of sample liquid/diluting water is regulated so that a constant residual ozone content or ozone consumption is regulated and that this mixing ratio is used for determining oxidisable substances contained in the water.

**10.** Method according to Claim 1, characterised in that when the mixing ratio of sample liquid/diluting water is kept constant, the quantity of ozone added is dosed so that a constant residual ozone content or ozone consumption is regulated and that this quantity of ozone added is used for determining oxidisable substances contained in the water.

**11.** Discontinuous method for determining oxidisable substances contained in the water of an aqueous sample liquid by oxidation of organic substances dissolved therein, the sample liquid being diluted in a dosed manner with diluting water and the determination of oxidisable substances contained in the water taking place in discrete individual measurements, characterised in that ozone is supplied to the sample liquid for oxidation, that after thorough mixing and oxidation, the residual ozone content is determined, that the addition of ozone takes place in a dosed quantity and that the time interval until the given constant residual ozone content is achieved, is determined and supplies a measurement of the oxidisable substances contained in the water.

**12.** Method according to Claim 4, characterised in that the redox potential is specified in the range of 300 to 800 mV.

**13.** Apparatus for carrying out the method according to one of Claims 1 to 12 with dosing pumps for the sample liquid and the diluting water, at least one reaction container, a mixing device, an oxidation device and a container outlet line, characterised in that the oxidation device is an ozone generator (7, 20) and that located in the container outlet line (11, 28) is an ozone measuring device (29) or a redox potential measuring cell (13).

**14.** Apparatus according to Claim 13, characterised in that the ozone generator (20) is connected to a reaction container (18), in which the diluting water is enriched with ozone.

**15.** Apparatus according to Claim 13, characterised in that the ozone measuring device (13) is connected to a regulating device (14) for the dosing pumps (1, 3) of the sample liquid and/or of the diluting water.

**16.** Apparatus according to Claim 15, characterised in that the ozone measuring device (13) is connected to a regulating device (14) for the ozone generator (7).

**17.** Apparatus for carrying out the method according to Claim 12 with a reaction container, a mixing device and an oxidation device, characterised in that the oxidation device is an ozone generator (7) with a dosing device and that the reaction container (2) is connected to an ozone measuring device (13).

**18.** Apparatus according to Claim 17, characterised in that connected to the reaction container (2) is a circuit line (15), in which a circulating pump (16) and the ozone measuring device (13) are located.

**19.** Apparatus according to Claim 17 or 18, characterised in that the dosing device of the oxidation device (7) and the ozone measuring device (13) are connected to an evaluation circuit (17), in which the time interval between the introduction of ozone and achieving a given value of the ozone content, of the ozone consumption or redox potential, is determined.

**Revendications**

**1.** Procédé continu pour la détermination des matières oxydables contenues dans l'eau, et en particulier de la demande chimique en oxygène (DCO) ou de la teneur en carbone organique (TOC - total organic carbon) du liquide d'un échantillon aqueux introduit en continu, par oxydation des matières organiques qui y sont dissoutes, le liquide de l'échantillon dosé étant dilué avec de l'eau de dilution, caractérisé en ce que le liquide de l'échantillon ne reçoit une injection d'ozone pour effectuer l'oxydation qu'après mélange et oxydation, que la teneur en ozone résiduel ou la consommation d'ozone est déterminée et que l'injection d'ozone et/ou le rapport de mélange liquide d'échantillon/eau de dilution est réglé ou sont réglés de façon à établir une teneur en ozone résiduel et une consommation d'ozone constantes prédéterminées.

**2.** Procédé selon la revendication 1, caractérisé en ce que le liquide de l'échantillon est dilué avec de l'eau de dilution avant l'injection d'ozone.

**3.** Procédé selon la revendication 1, caractérisé en ce que l'eau de dilution est enrichie en ozone et est ensuite mélangée au liquide de l'échantillon.

**4.** Procédé selon la revendication 1, caractérisé en ce que la teneur en ozone résiduel est déterminée par mesure du potentiel redox.

**5.** Procédé selon la revendication 1, caractérisé en ce que la consommation d'ozone est déterminée par mesure directe de l'ozone avant l'injection d'ozone et après l'oxydation et par calcul de la différence.

**6.** Procédé selon la revendication 1, caractérisé en ce qu'un rapport des substances oxydables contenues dans l'eau, et en particulier de la demande chimique en oxygène (DCO) à la quantité d'ozone injectée est réglée à 0,5 au maximum.

**7.** Procédé selon la revendication 3, caractérisé en ce que l'enrichissement en ozone de l'eau de dilution et l'oxydation s'effectuent dans deux enceintes de réaction séparées et qu'une mesure de l'ozone est effectuée chaque fois à la sortie des deux enceintes de réaction.

**8.** Procédé selon la revendication 1, caractérisé en ce que la détermination de la teneur en ozone résiduel est effectuée dans la gamme de 0,05 à 0,3 mg d'ozone par litre.

**9.** Procédé selon la revendication 1, caractérisé en ce que, en cas d'addition d'ozone maintenue constante, le rapport de mélange liquide de l'échantillon/eau de dilution est réglé de façon à établir une teneur en ozone résiduel ou une consommation d'ozone constante et que ce rapport de mélange est utilisé pour déterminer les substances oxydables contenues dans l'eau.

**10.** Procédé selon la revendication 1, caractérisé en ce que, en cas de rapport de mélange liquide d'échantillon/eau de dilution maintenu constant, la quantité d'ozone ajoutée est dosée de façon à établir une teneur en ozone résiduel ou une consommation d'ozone constante et que cette quantité d'ozone ajoutée est utilisée pour la détermination des substances oxydables contenues dans l'eau.

**11.** Procédé discontinu pour la détermination des substances oxydables contenues dans l'eau d'un liquide d'échantillon aqueux par oxydation des substances organiques qui y sont dissoutes, le liquide de l'échantillon dosé étant dilué avec de l'eau de dilution et la détermination des substances oxydables contenues dans l'eau s'effectuant par des mesures individuelles séparées, caractérisé en ce que le liquide de l'échantillon reçoit de l'ozone pour effectuer l'oxydation, qu'après mélange et oxydation la teneur en ozone résiduel est déterminée, que l'addition d'ozone s'effectue suivant une quantité dosée et que l'intervalle de temps requis pour atteindre une teneur en ozone résiduel constante prédéterminée est déterminée et fournit une mesure de la teneur en substances oxydantes contenues dans l'eau.

**12.** Procédé selon la revendication 4, caractérisé en ce que le potentiel redox est prédéterminé dans la gamme de 300 à 800 mV.

**13.** Dispositif pour mettre en oeuvre le procédé selon l'une des revendications 1 à 12, avec des pompes doseuses pour le liquide de l'échantillon et l'eau de dilution, au moins un récipient de réaction, un dispositif de mélange, un dispositif d'oxydation et une conduite d'évacuation du récipient, caractérisé en ce que le dispositif d'oxydation est un ozoniseur (7, 20) et qu'une cellule de mesure du potentiel redox (13) ou un dispositif de mesure de l'ozone (19) est monté dans la conduite d'évacuation du récipient (11, 28).

**14.** Dispositif selon la revendication 13, caractérisé en ce que l'ozoniseur (20) est connecté à un récipient de réaction (18) dans lequel l'eau de dilution est enrichie en ozone.

**15.** Dispositif selon la revendication 13, caractérisé en ce que le dispositif de mesure de l'ozone (13) est connecté à un dispositif de réglage (14) pour les pompes doseuses (1, 3) du liquide de l'échantillon et/ou de l'eau de dilution.

**16.** Dispositif pour mettre en oeuvre le procédé selon la revendiction 12, avec un récipient de réaction, un dispositif de mélange et un dispositif d'oxydation, caractérisé en ce que le dispositif d'oxydation est un ozoniseur (7) avec un dispositif de dosage et que le récipient de réaction (2) est connecté à un dispositif de mesure d'ozone (13).

**17.** Dispositif pour la mise en oeuvre du procédé de la revendication 12, avec un récipient de réaction, un dispositif de mélange et un dispositif d'oxydation, caractérisé en ce que le dispositif d'oxydation est un ozoniseur (7) comportant un dispositif de dosage et que le récipient de réaction (2) est connecté à un dispsitif de mesure de l'ozone (13).

18. Dispositif selon la revendication 17, caractérisé en ce qu'une conduite de circulation en circuit fermé (15) dans laquelle sont montés une pompe de circulation (16) et le dispositif de mesure de l'ozone (13) est raccordée au récipient de réaction 2.

19. Dispositif selon la revendication 17 ou 18, caractérisé en ce que le dispositif de dosage du dispositif d'oxydation (7) et le dispositif de mesure de l'ozone (13) sont connectés à un circuit d'évaluation (17) dans lequel est déterminé l'intervalle de temps entre l'introduction de l'ozone et l'obtention d'une valeur prédéterminée de la teneur en ozone, de la consommation d'ozone ou du potentiel redox.

FIG.1

FIG.2

FIG.3

14

FIG. 4

FIG. 5

15